Europäisches Patentamt

(19) European Patent Office    (11) Publication number: **0 079 962**

Office européen des brevets                                **B1**

(12) **EUROPEAN PATENT SPECIFICATION**

(45) Date of publication of patent specification: **28.08.85**    (51) Int. Cl.⁴: **G 01 N 33/546**

(21) Application number: **82902274.8**

(22) Date of filing: **28.05.82**

(86) International application number:
**PCT/US82/00737**

(87) International publication number:
**WO 82/04323 09.12.82 Gazette 82/29**

(54) **IMMUNOPRECIPITATION ASSAY.**

(30) Priority: **02.06.81 US 269727**

(43) Date of publication of application:
**01.06.83 Bulletin 83/22**

(45) Publication of the grant of the patent:
**28.08.85 Bulletin 85/35**

(84) Designated Contracting States:
**DE FR GB**

(56) References cited:
**US-A-3 690 834**
**US-A-3 839 153**
**US-A-3 850 752**
**US-A-3 879 262**
**US-A-3 976 763**
**US-A-4 150 949**
**US-A-4 262 089**
**US-A-4 302 438**
**US-A-4 305 720**
**US-A-4 305 925**
**US-A-4 308 026**

(73) Proprietor: **ELECTRO-NUCLEONICS, INC.**
**368 Passaic Avenue P.O. Box 803**
**Fairfield, NJ 07006 (US)**

(72) Inventor: **O'NEILL, Sean**
**5420 Roosevelt Street**
**Bethesda, MD 20034 (US)**
Inventor: **WU, Joseph**
**15032 Joshua Tree Road**
**Gaithersburg, MD 20760 (US)**

(74) Representative: **Barz, Peter, Dr.**
**Patentanwälte Dr. V. Schmied-Kowarzik Dipl.-**
**Ing. G. Dannenberg Dr. P. Weinhold Dr. D. Gudel**
**Dipl.-Ing. S. Schubert Dr. P. Barz**
**Siegfriedstrasse 8**
**D-8000 München 40 (DE)**

(56) References cited:
**JOURNAL OF IMMUNULOGICAL METHODS,**
**VOL. 29, No. 1, ISSUED SEPTEMBER 1979, T.**
**NISHIKAWA ET AL, 'COMPETITIVE**
**NEHPHELOMETRIC IMMUNOASSAY METHOD**
**FOR ANTIEPILEPTIC DRUGS IN PATIENT**
**BLOOD', PAGES 85-89**

R.F. RITCHIE, EDITOR, 'AUTOMATED IMMUNOANALYSIS', PART1,CHAPTER 15, J. GAULDIE ET AL, 'AUTOMATED NEPHELOMETRIC ANALYSIS OF HAPTENS', PAGES 321-333, PUBLISHED 1978, BY MARCEL DECKER, INC. (NEW YORK).

CHEMICAL ABSTRACTS, VOL. 94, No. 13, ISSUED 30 MARCH 1981 (COLUMBUS, OHIO, U.S.A.) M. HASHIMOTO, ABSTRACT OF JAPANESE PATENT 81 02, 554 ISSUING 12 JANUARY 1981, THE ABSTRACT No. 99302a, PAGE 390, COLUMN 2.

R.F. RITCHIE, EDITOR, 'AUTOMATED IMMUNOANALYSIS', PART 2, CHAPTER 19, R.J. ANDERSON ET AL, 'A RATE NEPHELOMETER FOR IMMUNOPRECIPITIN MEASUREMENT OF SPECIFIC SERUM PROTEINS', PAGES 409-469, PUBLISHED 1978, BY MARCEL DECKER, INC. (NEW YORK). SEE PAGES 409, 462 and 463

CLINICAL CHEMISTRY, VOL. 27, No. 3, ISSUED MARCH 1981, P.R. FINLEY ET AL., 'RATE-NEPHELOMETRIC INHIBITION IMMUNOASSAY OF PHENYTOIN AND PHENOBARBITAL', PAGES 405-409

JOURNAL OF IMMUNOLOGICAL METHODS, VOL. 38, ISSUED DECEMBER 1980, I. DEVERILL ET AL, 'LIGHT SCATTERING AND ABSORPTION-DEVELOPMENTS IN IMMUNOLOGY', PAGES 191-204.

CLINICA CHIMICA ACTA, VOL. 91, ISSUED 01 JANUARY 1979, T. NISHIKAWA ET AL, 'COMPETITIVE NEPHELOMETRIC IMMUNOASSAY OF THEOPHYLLINE IN PLASMA', PAGES 59-65.

# 0 079 962

**Description**

Background of the invention

Therapeutic monitoring of various agents present in serum and other body fluids is a rapidly growing segment of the clinical laboratory testing field. With therapeutically effective agents, the purpose of the monitoring is to indicate adjustments in dosage so as to maintain a patient at a therapeutically effective drug level. Below this level, the agent may be toxic. Such monitoring is, as will be recognized, important not only for following changes in levels of useful therapeutic agents, but also for ascertaining levels of a wide variety of physiologically active substances which may be present in body fluids including, for example, vitamins, enzymes and hormones.

Methods available for the fast and accurate quantitative or qualitative determination of biologically active substances, at low concentrations are limited in number. The physicians diagnosis of the patient or confirmation of the diagnosis frequently involves the detection and/or quantitation of one or more substances in body fluids. The ability to detect rapidly in body fluids the presence and amounts of such materials is often critical to the patient's life.

Several methods have, in the past, been used for the assay of body fluids, notably radioimmunoassay, thin layer chromatography, enzyme immunoassay and fluorescence immunoassay systems. The use of a radioimmunoassay technique suffers from several disadvantages among which are the hazards associated with or inherent in radioactive materials, associated handling problems, instability, the need for expensive equipment for the assays and the difficulties associated with the manipulation and separation of the free and bound forms of the radioactive materials. Thin layer chromatography is often quite slow in the development of the chromatogram, is sensitive to the presence of a variety of interfering factors and suffers from the lack of reproducibility or reliability. The use of enzyme or fluorescence immunoassay requires dealing with, and knowledge of very complicated chemistry involved in their preparation, storage and usage of the required reagents. Also, the method is sensitive to variations in environmental conditions, so that the methods have not proven entirely satisfactory to the users. Nephelometry has also been employed but the incubation times required have limited the practicality of the procedure, especially for automation. Additionally, the procedure requires the use of special equipment which is not routinely available in commercial laboratories. Additionally, unless the laser nephelometer is employed interference noise or background effects are so bothersome that the technique cannot be applied directly to serum. Additionally, they cannot be used at low wavelengths where one would obtain best sensitivity because of the unavoidable increase in output power which renders them dangerous. (Deverill and Reiner, Journal of Immunological Methods 38 (1980) 191—204, at page 195).

The invention

A procedure has now been discovered which substantially alleviates the problems aforesaid and makes possible the detection and accurate determination of small amounts of therapeutic drugs and other low molecular weight haptens in various body fluids and other samples. In accordance with the procedure, a competitive assay is set up for available antibody sites between the hapten, the concentration of which is to be determined and the same hapten conjugated to a carrier.

The process of the invention will be principally employed for the determination of hapten concentrations in mammalian serum. It is, however, applicable to the determination of hapten concentrations in a variety of liquid samples including, for example, human serum and plasma, urine, spinal fluid, amniotic fluid, etc.

The immunoprecipitation assay procedure of the invention may be applied to low molecular weight physiologically active substances, which for convenience are termed "haptens" in this description and claims. Haptens have been generally defined as protein free substances with chemical configurations such that they can react with specific antibodies but not such that they are capable of causing the formation of antibodies. For the purposes of this description, they include a wide variety of physiologically active materials, for example, antibiotics such as tetracycline, chloromycetin, streptomycin and aminoglycoside antibiotics such as amikacin, erythromycin, gentamicin, kanamycin, netilimicin, sisomicin, tobramycin and vancomycin; anti-epileptic drugs such as carbamazepine, dilantin (phenytoin), ethosuximide, phenobarbital, desipramine and imipramine; anti-arrythmics such as digoxin, digitoxin, propanolol, procainamide and disopyramide; alkaloids such as methadone, caffeine, dextromoramide, dipipanone, phenodoxone and darvone, catecholamines such as ephedrine, epinephrine and benzidine; abused drugs such as barbiturates, benzodiazepines such as chlordiazepoxide, diazapam, oxazepam, and marijuana; amino acids; steroids including estrogens, gestogens and androgens; vitamins; and sugars. Monitoring of the metabolites of these various compounds is also of interest.

For convenience, the compounds to which this invention is applicable will hereinafter be generically referred to as haptens or drugs. As the description of the invention proceeds, reference will be made to hapten-carrier conjugates or drug-carrier conjugates, and to the reaction products of the conjugates with antibodies.

Desirable therapeutic ranges for a number of haptens have been determined. Some of them are listed in Table I which should not be regarded as limiting with respect to the haptens to which this invention is applicable.

3

**0 079 962**

TABLE I

| Function | Drugs | Therapeutic concentration range | |
|---|---|---|---|
| Antibiotic | Gentamicin | 2—12 | µg/ml |
| | Tobramycin | 2—10 | µg/ml |
| | Amikacin | 5—40 | µg/ml |
| | Kanamycin | 5—40 | µg/ml |
| | Chloramphenicol | 2—10 | µg/ml |
| Anti-epileptics | Phenobarbital | 10—40 | µg/ml |
| | Theophylline | 10—20 | µg/ml |
| | Dilantin | 10—20 | µg/ml |
| | Primidone | 8—12 | µg/ml |
| | Ethosuximide | 40—80 | µg/ml |
| | Carbamazepine | 5—10 | µg/ml |
| | Valproate | 50—100 | µg/ml |
| Anti-depressives | Nortriptyline | 50—150 | µg/ml |
| | Desipramine | 150—250 | µg/ml |
| | Imipramine | 100—200 | µg/ml |
| Anti-arrythmic | Digoxin | 0.5—2.0 | µg/ml |
| | Digitoxin | 10—30 | µg/ml |
| | Propanolol | 50—100 | µg/ml |
| | Procainamide | 3—10 | µg/ml |
| | Disopyramide | 2—4 | µg/ml |

The specific compounds listed above and others, as is well known, all have desirable concentration ranges in which they exert their optimum therapeutic effects. Other physiologically active haptens from the above exemplary list do not have any desirable concentration. They are in fact toxic. However, it is often of critical importance for the physician to know the concentration of these materials in plasma or other body fluid so that appropriate life saving measures or detoxifications steps can be taken. Still others are not therapeutic in the sense that antibiotics are therapeutic, but may desirably be at a certain level in body fluids to maintain the health and continued well being of the subject. The concentration of certain other materials may be of interest to test for certain metabolic diseases or for pregnancy.

In general, as in the list given in Table I, the concentration ranges are very low and vary over extremely wide ranges. Desirable concentrations differ by orders of magnitude, for example, from micrograms per milliliter to nanograms per milliliter.

For convenience, all concentration ranges to which the process of this invention applies, whether they be therapeutic ranges, toxic ranges, ranges which will help to maintain good health, test ranges or other ranges will be referred to herein as "concentration of ranges of interest".

The method of assay of this invention is based on competitive immunological reactions in which the reactants and all but one of the products are soluble in the test medium. Specifically the hapten, the hapten-carrier conjugate, the antibody and the hapten-antibody reaction product are soluble in the test medium, and the conjugate-antibody is not. Therefore, as more and more of the last mentioned reaction product forms, the turbidity of the test medium increases. To the extent that the hapten reacts with antibody, the turbidity will decrease.

The foregoing may be summarized as follows:

| HAPTEN | HAPTEN-CARRIER |
|---|---|
| Soluble | Soluble |

$$+$$
ANTIBODY

Soluble

| HAPTEN-CARRIER-ANTIBODY | HAPTEN-ANTIBODY |
|---|---|
| Insoluble, turbidity increases | Soluble, turbidity decreases |

In the competitive assay, the soluble hapten present in the patient's serum or other body fluid competes with the soluble hapten-carrier (conjugate) for a limited number of available antibody sites thereby reducing the amount of insoluble conjugate-antibody complex which will form and concurrently

4

decreasing the turbidity of the solution. The degree of turbidity, therefore, is inversely proportional to the concentration of free hapten in a test medium containing free hapten together with hapten-carrier, antibody, conjugate-antibody and hapten-antibody.

The first necessity for conducting the competitive assays of this invention is the preparation of a series of hapten-carrier conjugates of different hapten or drug to immunogenic carrier ratio.

As used herein, the term "immunogenic carrier" is meant to include those materials which have the property of independently eliciting an immunogenic response in a host animal when injected therein, and can be directly or indirectly coupled by covalent bonding to the hapten. Suitable materials include naturally occurring proteins, natural and synthetic polymeric compounds such as polypeptides, polysaccharides, polystyrene, polyacrylates and the like. Normally, for use in this invention, the molecular weight of the carrier will be at least 60,000 Daltons.

For the preparation of the conjugate, there may be functional groups already present on the hapten and on the carrier. These may react directly to form conjugates. These groups include, for example, hydroxyl, amino and carboxyl groups. If no suitable functional group is present, it may be put in place. For example, with certain steroid haptens it may be possible to substitute an hydroxyl group on the molecule by fermentation procedures, or to monoesterify a hydroxyl group with a dicarboxylic acid to leave a free carboxyl group.

Other reagents may be employed to effect coupling between hapten and carrier. These include, for example, dialdehydes such as glutaric aldehyde; hydrazines; dinitrodiphenylsulfone, diisocyanates such as toluenediisocyanate; di- or tri-chlorotriazines; carbodiimides; cyanogen bromide and diazocompounds.

Procedures for forming conjugates by any of the methods suggested above will be familiar to those skilled in the art.

Since the carrier compound will normally carry a large number of functional groups, it is apparent that, within reason, any selected number of hapten molecules can be joined to a particular carrier. Thus, the ahpten-carrier ratio may vary from as low as 1:1 to as high as 100:1 or even higher. An important aspect of this invention is the discovery that when decreasing optical density at wave lengths from 280—600 nm is plotted as the ordinate against increasing concentration of free hapten in media containing conjugate, free hapten, hapten-conjugate, antibody and hapten-antibody, the shape of the curve will vary with changing drug:carrier ratios at which the concentration of free hapten can be obtained with optimum sensitivity.

It will be apparent from the above that the number of hapten molecules attached to each molecule or carrier has an important effect on the use of turbidity measurements as a method of immunoassay. At low ratios of hapten to carrier, e.g. 3:1, the conjugate will not produce sufficient turbidity to be of practical use as a measuring tool. At the other extreme of high hapten to carrier ratio, e.g. 50:1, the hapten-conjugate-antibody reaction will produce too much turbidity to allow sensitive measurements of changes in turbidity.

Sensitivity to changes in free hapten concentration will be very poor at both ends of the scale.

This invention will be better understood by reference to the figures in which:

Fig. 1 is a graph of standard curves in which optical density of separate mixtures containing fixed amounts of conjugate and soluble antibody to the conjugate is plotted against increasing hapten concentration, the conjugate being Dilantin-human serum albumin (HSA).

Fig. 2 is similar to Fig. 1 except that the conjugate is gentamicin-HSA.

Fig. 3 is similar to Fig. 1 except with a smaller number of curves and the conjugate is phenobarbital-HSA.

Fig. 4 is similar to Fig. 1 except with a smaller number of curves and the conjugate is theophylline-HSA.

Fig. 5 is similar to Fig. 1 except with a smaller number of curves and the conjugate is tobramycin-HSA.

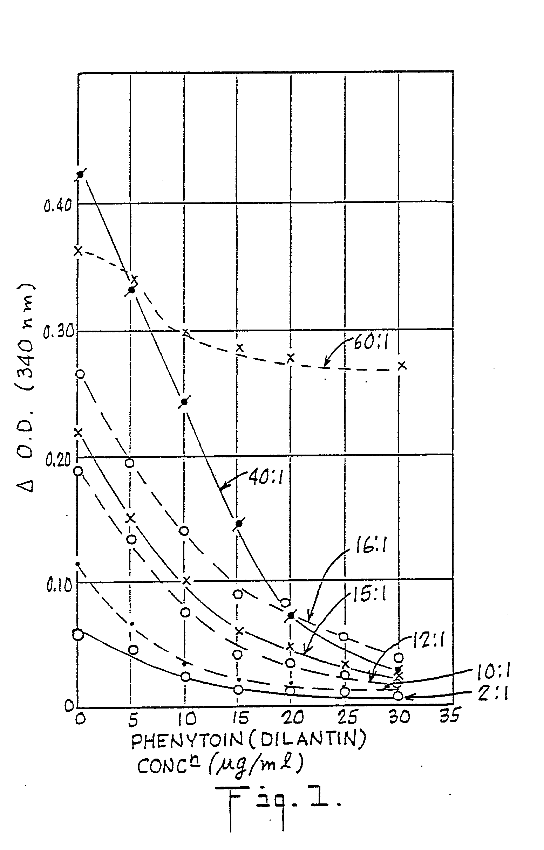

Referring to Table I, it will be seen that the concentration range of interest for Dilantin is 10—20 µg/ml. Referring to Fig. 1, it will be seen that over the hapten concentration range of from 10—20 µg/ml, the optical density change is relatively small when the hapten to carrier ratio is low, e.g. 6:1 or 10:, and that the same is true at a high ratio, e.g. 60:1. However, if the hapten to carrier ratio is from 12:1 to 40:1, there is a relatively large change in optical density with relatively small changes in hapten concentration. This is the region of optimum sensitivity. It is the region in which the average slope of the curve is such that there is sufficient decrease in optical density with increase in hapten concentrations so that there is optimum response in optical density change to change in concentration. As shown in Fig. 1, the average slopes of the curves in the Dilantin concentration range from 10 to 20 µg/ml at dilantin-HSA ratios from 12:1 to 40:1 is from about 30° to 60°. It will be known, however, that if the scale of the graph is changed, the average slope will also change so that it is difficult to assign specific values to the average slope of optimum sensitivity. It will be apparent, however, that no matter what scale is selected, the graph will show a range of dilantin-HSA ratios where there is optimum sensitivity and this will be readily recognized by the shape of the curve.

Still another explanation of optimum sensitivity is based on the recognition that sensitivity is at a minimum at both high and low hapten-carrier ratios and passes through a maximum, so that a curve plotting sensitivity against hapten-carrier ratio may start at a low point at the lowest hapten-carrier ratio pass, through a maximum as the hapten carrier ratio increases, and then return to the low value with increasing hapten-carrier ratio. The basis of this invention is the discovery that for the hapten, the concentration of which is to be determined, there is a range of hapten-carrier ratios where optimum sensitivity is achieved in the concentration range of interest.

Maximum sensitivity is, of course, the ability to recognize minimum changes in hapten concentration.

As a practical matter, it is not essential to obtain maximum sensitivity to achieve useful measurements. The sensitivity should, however, be as close to maximum as is reasonably possible. More specifically, it should be what is characterized herein as "optimum sensitivity". To quantize the sensitivity, it is reasonable to say that there is optimum sensitivity when there is an ability to recognize concentration changes of ±5%.

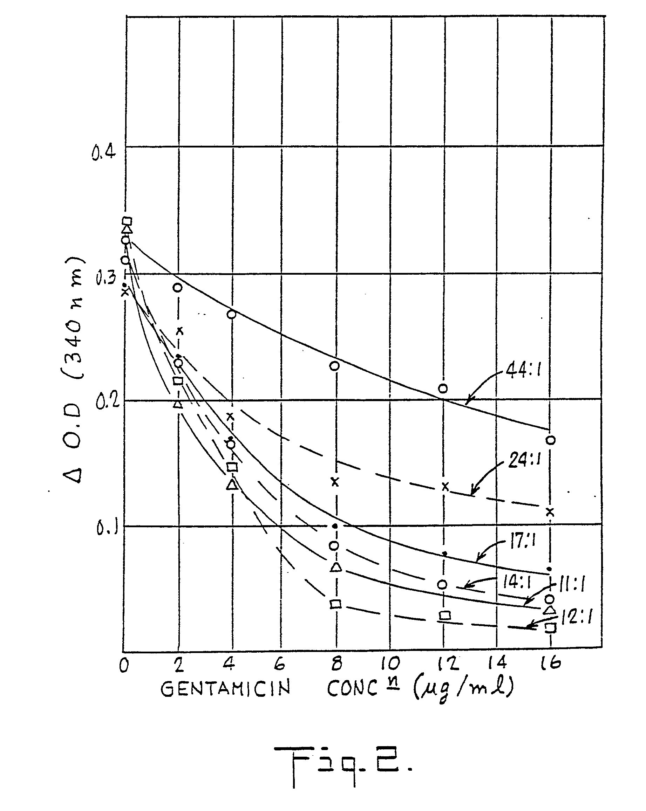

Fig. 2 is similar to Fig. 1 except that the drug under investigation is the antibiotic gentamicin. The therapeutic drug range is 2 to 12 μg/ml. It will be seen that at low drug:carrier ratios, e.g. 11:1, 12:1 or 14:1, there is relatively little change in optical density over the range of 8 to 16 μg/ml. An acceptable assay system must provide the possibility of optimum sensitivity in this range since it overlaps the therapeutic range. At high drug:carrier ratio e.g. 44:1, discrimination or sensitivity is not good along the entire length of the standard curve.

Intermediate ratios of 17:1 or 24:1 provide the opportunity for optimum sensitivity over all of the required assay range for this antibiotic.

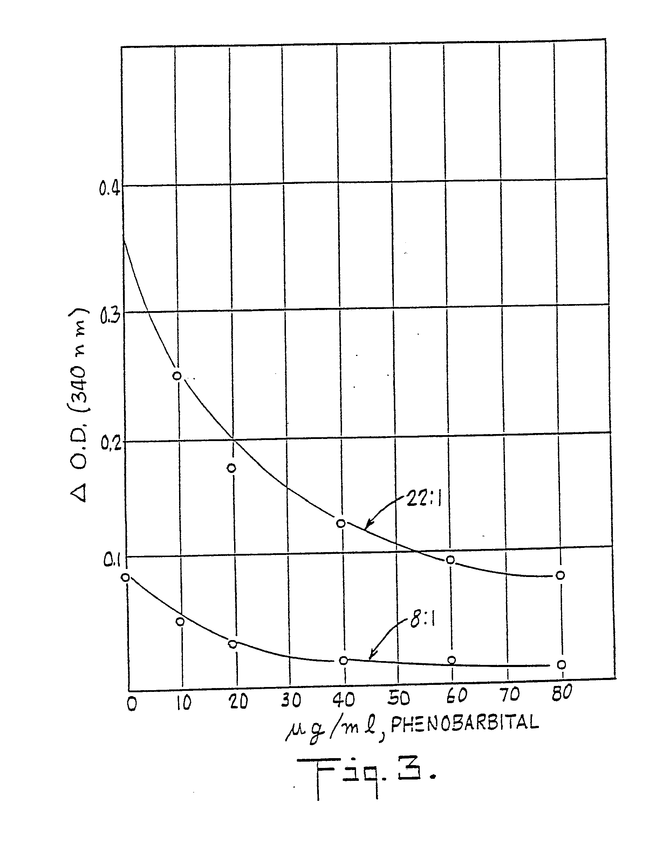

Fig. 3 shows only two curves. Curve A is phenobartital-HSA ratio of 8:1. Curve B is at a ratio of 22:1. It will be seen that at a ratio of 8:1 the change in optical density at the concentration range of interest (10—40 μg/ml) is very low, but at a ratio of 22:1 the change is very marked. It is, in fact, sufficiently great so that phenobarbital concentrations can be obtained as described herein. In fact, ratios of from 15:1 to 30:1 will permit the determination of optimum sensitivity.

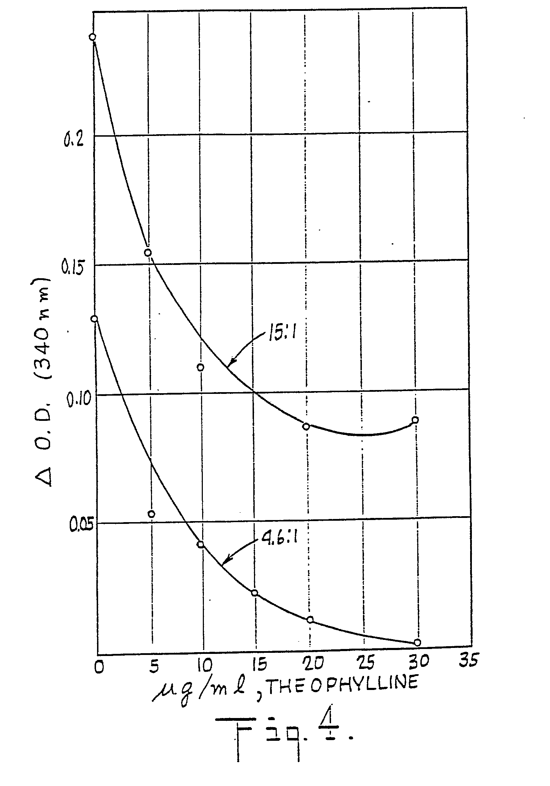

Fig. 4 is similar to Fig. 3. Curve D shows that with theophylline a hapten:carrier ratio of 4.6:1 will not permit determination of optimum sensitivity at the range of interest because the optical density is too low. There is not sufficient turbidity. At a concentration of 10 μg/ml the optical density is only 0.04. At 20 μg/ml it is only 0.02. However, at a ratio of 15: not only is the optical density sufficiently high, but also the change in optical density over the concentration range of interest is sufficiently high to permit accurate measurements. For theophylline the useful hapten:carrier range is from about 10:1 to 20:1.

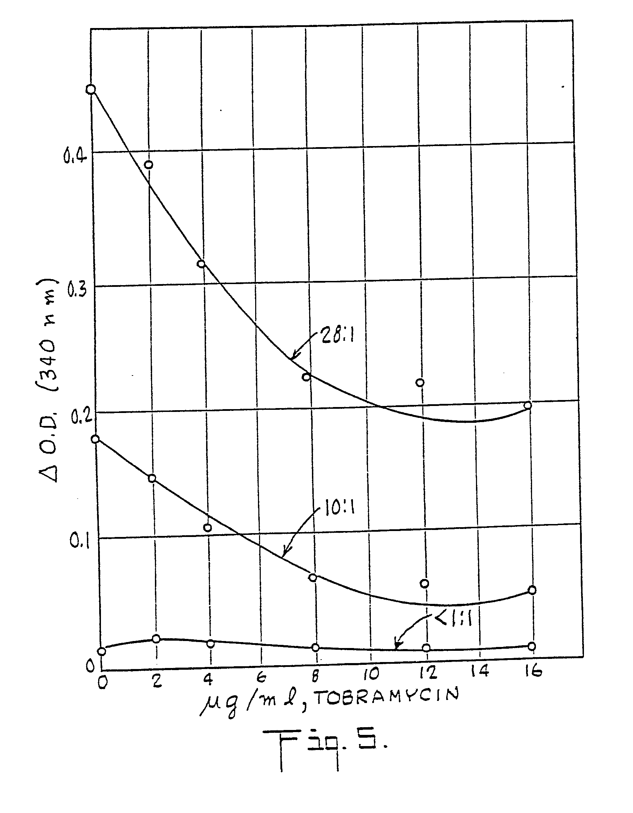

Fig. 5 shows that with tobramycin-HSA at ratios of under 1:1 and 10:1 the optical densities are too low for useful measurements and the changes in optical densities are not sufficient in the concentration range of interest, i.e. 2 to 10 μg/ml. However, at 28:1 the optical density and change in optical density is acceptable. It was found that tobramycin-HSA ratios of 30:1 were not attainable. The useful ratios for this antibiotic are from 20:1 to 28:1.

It sometimes happens that at high ratios the hapten-carrier conjugate is insoluble. Clearly such conjugates are not useful for the practice of this invention.

As stated above, the preparation of the conjugate in which the hapten is covalently bound to the immunogenic carrier is in accordance with techniques well known to those skilled in the art. The drug:carrier ratio will normally depend upon the amount of drug or hapten employed. For high ratios of drug to carrier, normally high concentrations of drugs will be employed. The course of the reaction and the determination of the drug:carrier ratio of the final product can be determined by any of a number of methods, for example, ultraviolet spectrophotometry, radiolabeling and the like. Suitable illustrations of the synthesis of conjugates with various ratios are given in the examples.

Anti-sera containing the antibody to the hapten-carrier conjugate may be raised in any convenient mammal, for example, goat, sheep, horse or rabbit or by other standard procedures including the hybridoma technique. The hapten-carrier ratio in the conjugate used to raise the antibody is not critical. Any of the conjugates synthesized for the preparation of the standard curves may be employed. The conjugate mixed with an adjuvant, for example, Freund's adjuvant is injected into the selected animal and the animal is bled in about one month. The serum containing the antibody is separated from the whole blood.

It is preferred, but not essential, that the carrier used in the conjugate for the preparation of the antisera be different from the carrier employed in the conjugate used in the preparation of standard curves. The use of different carriers limits the possibility of cross reactions. For example, HSA can be used in the conjugates for the standard curves, and bovine serum albumin (BSA) can be used to prepare antisera.

A particular advantage of the procedure of this invention is that by selecting the correct drug:carrier ratio it is possible to determine the optical density in the ultraviolet-visible region of the spectrum at a wavelength of from 280—600 nm. At these wavelengths, there is improved sensitivity compared to the sensitivity which can be attained at higher wavelengths, the techniques are generally less difficult and the available instruments, such as the spectrophotometer, Centrifugal Analyzer, and Clinical Chemistry Analyzer are relatively inexpensive, easy to use and readily adaptable to automation. The procedures are sufficiently rapid so that they are readily adaptable to automation techniques.

To achieve optimum sensitivity measurements, it is best to use concentrations of antisera and conjugate which give maximum absorbance at the selected wavelength. These concentrations are readily determined by standard measurements. To make the determinations, antisera raised by the conjugate is adjusted by serial dilution to values of, say 1:1, 1:10, 1:20—1:160, and samples of such dilution are allowed to react with similarly diluted conjugate. The optical density is determined for each reaction at a selected wavelength from 280 to 600 nm. The wavelength will be the same wavelength as is used when preparing the standard curves. These measurements are made for a series of high to low drug:carrier ratios. There will be an optimum absorbance for each drug:carrier ratio and each antisera dilution. For instance, the optimum absorbance for gentamicin-HSA conjugate with a 1:50 dilution of antisera is 1:10.

The reactions are carried out in aqueous media which ideally, but not necessarily, will be the same media employed to prepare the standard curves and to determine the concentration of the hapten in a sample. The medium may contain about 2 to 5% polyethylene glycol (molecular weight 40,000 to 80,000, preferably about 60,000) as an accelerator and in some instances, up to about 10% of a water miscible polar organic solvent to aid solubility. These include alcohols, ketones and amides, for example, methyl or ethyl alcohol, acetone and dimethylformamide.

The pH of the medium will normally be from about 3 to 11, preferably 6 to 9. Various buffers together with sufficient dissolved salt to be isotonic may be employed. Illustrative buffers include carbonate, phosphate, borate, Tris, barbital and the like. The buffer employed is not critical to the present invention, but in particular measurements, one buffer may be preferred over another.

Moderate temperatures are normally employed for carrying out the measurements and usually constant temperatures during the period of assay is employed. The temperatures typically range from about 20°C to 45°C, more usually from about 25°C to 40°C.

Once the conditions for maximum absorbance have been determined, it is possible to set up the competitive immunoassay by mixing the sample to be determined with the selected dilution of antisera and conjugate. The first assay is with known concentrations of hapten sample in human plasma or other body fluid. The competitive immunoassay is followed by plotting optical density against hapten concentration. From these standard curves, it is possible to select a conjugate with a drug:carrier ratio which will provide optimum sensitivity in the concentration range of interest. Once the standard curves have been prepared and the best drug:carrier ratio determined, it is possible to conduct competitive assays using samples with unknown concentrations of hapten. Typically, these samples will be human serum containing unknown concentrations of drugs. The optical density of the mixture containing the conjugate at the optimum dilution, the antisera at the optimum dilution and the unknown concentration of hapten will be compared with the standard curves to identify the concentration of hapten in the serum sample.

In summary, the process of this invention comprises as applied to the determination of haptens in blood, the steps of:

1. Preparing a series of drug:carrier conjugates at different drug-carrier ratios. These will be prepared in aqueous media and will normally contain from 2—10 mg protein/ml.

2. Preparation of antibody, for example, by use of a conjugate to raise antisera in a mammal.

3. Collect the antisera and separate the serum.

4. Determine the conditions for maximum absorbance at a selected wavelength for each antisera and each conjugate by comparison of optical densities.

5. Prepare standard curves by plotting the change in optical density against the change in hapten concentration for a variety of conjugates with different drugs:carrier ratios to select the drug:carrier ratio which gives optimum sensitivity over the hapten range of interest.

6. Determining the concentration of hapten in an unknown sample by mixing the sample with antisera and the selected drug:carrier conjugates and determining the optical density.

7. Compare the optical density thus obtained with the optical density of known samples as shown by the standard curves to determine the concentration of hapten in the sample measured.

With samples other than blood serum, there will be appropriate modifications in the process outlined above.

Test kits may be made available for the convenient practice of this invention. The kits will contain the ingredients to prepare standard curves and may contain sufficient buffered liquid to utilize as the reaction solvent. The kits, therefore, will contain hapten at various standard concentrations, antisera to the hapten, and conjugate dissolved in the test media solvent. The drug:carrier ratio in the conjugate will be at a selected value to provide optimum sensitivity, and the antiser and conjugate solutions will be at proper dilutions to provide optimum absorbance as described above. Clinical laboratories which in the course of a day will conduct a large number of determinations for the same hapten will not need to prepare standard curves for each determination. A test kit for such laboratories may omit the samples of hapten at standard concentrations, provided that at least one set of such samples is available.

A specific advantage of the process of this invention is the high in day and between day precision of the assays. The concentration values for aliquots of the same sample measured on the same day or on successive days are measurable with a degree of precision which is consistent with good medical practice.

The following non-limiting examples are given by way of illustration only.

Example I

Determination of gentamicin in serum

I. The formation of a gentamicin-human serum albumin conjugate is achieved as follows:

A. To a solution of 100 mg of human serum albumin in 10 ml 0.01 M phosphate buffer pH 7.4 containing 0.15 M NaCl, 150 mg of gentamicin sulfate in 8 ml of distilled water containing 2 ml $^{125}$I-gentamicin as tracer (approximately 600 cpm/ml) are added with stirring. To the mixture is added portionswise with stirring 1 mg of 1-ethyl-3-(3-dimethylaminopropyl) carbodiimide HCl over a period of 20 minutes. The resulting reaction mixture is stirred at room temperature for another 20 minutes and the excess condensing agent and small molecules are then removed by dialyzing against 0.01 M phosphate buffer containing 0.15 M NaCl pH 7.4 at 4°C. The ratio of the resulting conjugate is determined from the $^{125}$I-tracer to be 17 molecules of gentamicin per molecule of human serum albumin.

B. When the procedure A is repeated omitting the $^{125}$I-gentamicin tracer, the conjugates are substantially identical in properties.

C. When the procedure A is repeated using the following quantities and reaction conditions, 100 mg human serum albumin; 325 mg gentamicin sulfate; 2 mg 1-ethyl-3-(3-dimethylaminopropyl) carbodiimide HCl; same amount of $^{125}$I-gentamicin tracer; reaction time: One hour at room temperature and 24 hours at 4°C, the ratio of the resulting conjugate is 44 molecules of gentamicin per molecule of human serum albumin.

D. When the procedure A is repeated using the following quantities and reaction conditions, 100 mg human serum albumin; 200 mg gentamicin sulfate; 1.5 mg 1-ethyl-3(3-dimethylaminopropyl)carbodiimide HCl; same amount of $^{125}$I-gentamicin tracer; reaction time, 30 minutes at room temperature, the ratio of the resulting conjugate is 24 molecules of gentamicin per molecule of human serum albumin.

II. Determination of the equivalent point of gentamicin per human serum albumin conjugate bound to gentamicin antibody.

A series of dilution of gentamicin conjugate (the ratio of gentamicin/albumin, 17) is titrated by an appropriate dilution of gentamicin antibody in an aqueous diluting agent which consists of 3% polyethyleneglycol (molecular weight 60,000), 0.25 M NaF and 0.15 M NaCl, pH 7.5, yielding a Gaussian type of curve. A typical titration result after 30 minutes incubation at room temperature is obtained at 340 nm, from a spectrophotometer and tabulated in Table I.

Table I. Data obtained from the titration of a series of dilution of gentamicin-human serum albumin conjugate (0.01 ml) with a dilution of 1:50 gentamicin antibody.

| Gentamicin conjugate dilution | Absorbance |
| --- | --- |
| Undiluted (4 mg protein/ml) | 0.371 |
| 1:2 | 0.530 |
| 1:5 | 0.705 |
| 1:10 | 0.718 |
| 1:20 | 0.481 |
| 1:40 | 0.276 |
| 1:80 | 0.149 |
| 1:160 | 0.086 |

From the titration, the dilution yielding the maximum absorbance (1:10) is used to establish the ratio of antiserum and gentamicin conjugate as the reagents for the construction of a standard curve.

III. Construction of a gentamicin standard curve and determination of unknown patient samples.

Standard solutions are prepared from gentamicin dissolved in gentamicin free normal human serum at concentrations ranging from 2 to 16 µg/ml. The procedure for the assay is as follows: A disk containing 20 numbered wells is positioned on a supporter (disks and supporter are manufactured by Electro-Neucleonics, Inc.). Each well has two compartments: sample compartment and reagent compartment. A 10 µl of gentamicin conjugate (1:10 dilution) and a 50 µl of various standard solutions (or patient samples) are pipetted into each sample compartment. A 0.50 ml of gentamicin antibody (1:50 dilution) is pipetted into each reagent compartment. The disk is fitted into an instrument Gemeni™ (Electro-Neucleonics, Inc.) operated by a specific computer card, giving 4 minutes incubation at 37°C and 3 minutes mixing and reaction. A standardization curve is constructed by subtracting the initial 6 seconds reaction absorbance reading from the 3 minutes reaction absorbance reading for each sample. This plot of data is shown in Fig. 2.

Assay of gentimicin in 11 patients' samples by the present assay and, for comparison, an enzyme immunoassay gives, excluding two severely lipemic samples from the serum patient, a regression equation,

$$Y=0.87X+0.11$$

with the correlation coefficient, 0.973.

The precision of this assay is tested by assaying two control sera within-day assay and three control sera for between-day assay as shown in Table II.

### TABLE II
#### Precision of gentamicin assay

| | N | X μg/ml | SD μg/ml | CV% |
|---|---|---|---|---|
| Within-day | | | | |
| Control 1 | 10 | 1.55 | 0.36 | 23.3 |
| Control 2 | 21 | 6.90 | 0.53 | 7.5 |
| Between-day | | | | |
| Control 1 | 6 | 3.19 | 0.76 | 23.5 |
| Control 2 | 6 | 5.10 | 0.50 | 9.8 |
| Control 3 | 6 | 10.30 | 1.10 | 10.6 |

As expected, the precision is best for the control value in the mid-range of the standard curve, where the therapeutic concentration is monitoring, i.e. 4—8 μg/ml.

Example II
Determination of dilantin in serum

I. The formation of a dilantin-human serum albumin is achieved as follows:

To a solution of 40 mg of dilantin valeric acid (valeric acid derivative of dilantin) in 6 ml of a 1:5 mixture of pyridine:water, 40 mg of 1-ethyl-3-(3-dimethylaminopropyl) carbodiimide HCl are added with stirring and followed with 100 mg human serum albumin in 4 ml distilled water. The solution is stirred at room temperature for 30 minutes and then 20 mg more of 1-ethyl-3-(3-dimethylaminopropyl) carbodiimide HCl are added. The resulting reaction mixture is stirred at room temperature for $1\frac{1}{2}$ hour and then dialyzed against 0.01 M phosphate buffer containing 0.15 M NaCl, pH 7.4 at 4°C. The ratio of the resulting conjugate analyzed spectrophotometrically results to be about 40.

II. Determination of the equivalent point of dilantin-human serum albumin conjugate bound to be the dilantin antibody.

A series of dilution of dilantin conjugate (the ratio of dilantin/albumin being 40) is titrated by an appropriate dilution of dilantin antibody in an aqueous diluting agent which consists of 3% polyethylene glycol (molecular weight, 60,000), 0.25 M NaF and 0.15 M NaCl, pH 7.6, yielding a Gaussian type of curve. A typical titration result after 30 minutes incubation at room temperature is obtained at 340 nm from a spectrophotometer and tabulated in Table III.

Table III. Data obtained from the titration of a series of dilutions of dilantin-human serum albumin conjugate (0.01 ml) with a dilution of 1:50 dilantin antibody (0.50 ml).

### TABLE III

| Dilantin conjugate dilution | Absorbance |
|---|---|
| Protein Undiluted (7.7 mg/ml) | 0.148 |
| 1:2 | 0.182 |
| 1:5 | 0.304 |
| 1:10 | 0.532 |
| 1:20 | 0.637 |
| 1:40 | 0.401 |
| 1:80 | 0.255 |
| 1:160 | 0.182 |
| 1:320 | 0.145 |

From the titration, the dilution yielding the maximum absorbance (1:20) is used to establish the ratio of antiserum and dilantin conjugate as the reagents for the construction of a dilantin standard curve.

III. Construction of a dilantin standard curve and determination of unknown patient samples.

Standard solutions are prepared from dilantin stock solution which is then mixed with dilantin-free normal human serum, giving the desired concentration in the range of 5 to 30 µg/ml. The dilantin stock solution is prepared by dissolving the desired amount of dilantin in a small amount of ethylalcohol and then diluted with distilled water to the mark of a volumetric flask. The procedure for the assay is as follows: A disk containing 20 numbered walls is positioned on a supporter (disks and supporter are manufactured by Electro-Nucleonics, Inc.). Each well has two compartments: Sample compartment and reagent compartment. A 10 µl of dilantin conjugate (1:20 dilution) and a 10 µl of various standard solutions (or patients' samples) are pipetted into each sample compartment. A 0.50 ml of dilantin antibody (1:50 dilution) is pipetted into each reagent compartment. The disk is fitted into an instrument such as a centrifugal analyzer (for example: Genemi (Electro-Nucleonics, Inc., Fairfield, NJ) operated by a specific computer card, giving 4 minutes incubation at 37°C and 3 minutes mixing and reaction. A standardization curve is constructed by subtracting the initial 6 seconds reaction absorbance reading from the 3 minutes reaction absorbance reading for each sample. This plot of data is shown in Fig. 1.

IV. Precision and accuracy for clinical assay

Assay of dilantin in 45 patients' samples by the present assay and enzyme immunoassay gives a regression equation,

$$Y = 1.12X + 0.24$$

with the correlation coefficient, 0.963.

The precision of this assay is tested by assaying two control sera for within-day assay and five control sera for between-day assay as shown in Table IV.

TABLE IV
Precision of dilantin assay

| | N | X | SD | CV% |
|---|---|---|---|---|
| | | µg/ml | | |
| Within-day | | | | |
| Control 1 | 24 | 11.40 | 0.53 | 4.6 |
| Control 2 | 24 | 16.60 | 1.08 | 6.5 |
| Between-day | | | | |
| Control 1 | 10 | 3.86 | 0.93 | 24.0 |
| Control 2 | 10 | 6.60 | 0.77 | 11.7 |
| Control 3 | 10 | 7.60 | 0.55 | 7.2 |
| Control 4 | 10 | 12.05 | 0.79 | 6.6 |
| Control 5 | 10 | 17.86 | 1.70 | 9.7 |

As expected, the precision is best for the control value in the mid-range of the standard curve, where the therapeutic concentration is monitoring, i.e., 10—20 µg/ml.

**Claims**

1. A method of measuring the amount of free hapten in a sample and involving a competitive immunoprecipitation assay, said method comprising the steps of forming a solution that includes said free hapten and a hapten-carrier conjugate and antibody which will bind with said free hapten and with said conjugate, in which the turbidity of said solution varies to a measurable degree dependent upon the amount of free hapten and the ratio of hapten to carrier in said conjugate in said solution, and determining said solution turbidity by analysis of radiation at a wavelength within the range 280—600 nm directly transmitted through said solution.

2. A method as in claim 1 wherein the conjugate is gentamicin covalently bound to human serum albumin in a gentamicin to albumin ratio of from 17:1 to 24:1.

3. A method as in claim 1 wherein the conjugate is phenytoin covalently bound to human serum albumin in a phenytoin to albumin ratio of from 12:1 to 40:1.

4. A method as in claim 1 wherein the conjugate is phenobarbital covalently bound to human serum albumin in a phenobarbital to albumin ratio of from 15:1 to 30:1.

10

5. A method as in claim 1 wherein the conjugate is theophylline covalently bound to human serum albumin in a theophylline to albumin ratio of from 10:1 to 20:1.

6. A method as in claim 1 wherein the conjugate is tobramycin covalently bound to human serum albumin in a tobramycin to albumin ratio of from 20:1 to 28:1.

7. A hapten-carrier conjugate useful in completing a competitive immunoprecipitation assay to determine the amount of free hapten in a sample comprising a hapten-carrier conjugate having a hapten to carrier ratio such that, when said conjugate is mixed in solution with said free hapten and an antibody that will bind with said free hapten and with said conjugate, varying amounts of free hapten will produce changes in turbidity of said solution that are determinable by measuring radiation within a wavelength range of 280—600 nm directly transmitted through said solution.

8. A hapten-carrier conjugate as in claim 7 wherein the conjugate is gentamicin covalently bound to human serum albumin in a gentamicin to albumin ratio of from 17:1 to 24:1.

9. A hapten-carrier conjugate as in claim 7 wherein the conjugate is phenytoin covalently bound to human serum albumin in a phenytoin to albumin ratio of from 12:1 to 40:1.

10. A hapten-carrier conjugate as in claim 7 wherein the conjugate is phenobarbital covalently bound to human serum albumin in a phenobarbital to albumin ratio of from 15:1 to 30:1.

11. A hapten-carrier conjugate as in claim 7 wherein the conjugate is theophylline covalently bound to human serum albumin in a theophylline to albumin ratio of from 10:1 to 20:1.

12. A hapten-carrier conjugate as in claim 7 wherein the conjugate is tobramycin covalently bound to human serum albumin in a tobramycin to albumin ratio of from 20:1 to 28:1.

**Revendications**

1. Procédé permettant de mesurer la concentration en haptène libre dans un prélèvement comportant un dosage compétitif par immunoprécipitation, caractérisé en ce qu'il comprend les étapes de préparation d'une solution contenant ledit haptène libre, un conjugué haptène-support et un anticorps se liant audit haptène libre et audit conjugué, la turbidié de ladite solution variant à un degré mesurable dépendant de la concentration d'haptène libre dans ladite solution et du rapport haptène-support dans ledit conjugué, et de mesure de la turbidité de ladite solution par analyse de radiation à une longueur d'onde comprise entre 280 et 600 nm transmise directement par ladite solution.

2. Procédé selon la revendication 1, caractérisé en ce que le conjugué est la gentamicine liée de façon covalente à l'albumine de sérum humain, le gentamicine et l'albumine étant dans un rapport compris entre 17:1 et 24:1.

3. Procédé selon la revendication 1, caractérisé en ce que le conjugué est la phénytoïne liée de façon covalente à l'albumine de sérum humain, la phénytoïne et l'abumine étant dans un rapport compris entre 12:1 et 40:1.

4. Procédé selon la revendication 1, caractérisé en ce que le conjugué est le phénobarbital lié de façon covalente à l'albumine de sérum humain, le phénobarbital et l'albumine étant dans un rapport compris entre 15:1 et 30:1.

5. Procédé selon la revendication 1, caractérisé en ce que le conjugué est la théophylline liée de façon covalente à l'albumine de sérum humain, la théophylline et l'albumine étant dans un rapport compris entre 10:1 et 20:1.

6. Procédé selon la revendication 1, caractérisé en ce que le conjugué est la tobramycine liée de façon covalente à l'albumine de sérum humain, la tobramycine et l'albumine étant dans un rapport compris entre 20:1 et 28:1.

7. Conjugué haptène-support utile pour réaliser un dosage par immunoprécipitation compétitif en vue de déterminer la concentration en haptène libre dans un prélèvement, caractérisé en ce que le conjugué haptène-support présente un rapport haptène/support tel que, lorsque ledit conjugué est mélangé en solution avec ledit haptène libre et un anticorps se liant audit haptène libre et audit conjugué, des variations de la concentration en haptène libre produisent des variations de la turbidité de ladite solution déterminables par mesure de la radiation dans un intervalle de longueurs d'onde compris entre 280 et 600 nm directement transmis par ladite solution.

8. Conjugué haptène-support selon la revendication 7, caractérisé en ce que le conjugué est la gentamicine liée de façon covalente à l'albumin de sérum humain, la gentamicine et l'albumine étant dans un rapport compris entre 17:1 et 24:1.

9. Conjugué haptène-support selon la revendication 7, caractérisé en ce que le conjugué est la phénytoïne liée de façon covalente à l'albumine de sérum humain, la phénytoïne et l'albumine étant dans un rapport compris entre 12:1 et 40:1.

10. Conjugué haptène-support selon la revendication 7, caractérisé en ce que le conjugué est le phénobarbital lié de façon covalente à l'albumine de sérum humain, le phénobarbital et l'albumine étant dans un rapport compris entre 15:1 et 30:1.

11. Conjugué haptène-support selon la revendication 7, caractérisé en ce que le conjugué est la théophylline liée de façon covalente à l'albumine de sérum humain, la théophylline et l'albumine étant dans un rapport compris entre 10:1 et 20:1.

11

# 0 079 962

12. Conjugué haptène-support selon la revendication 7, caractérisé en ce que le conjugué est la tobramycine liée de façon covalente à l'albumine de sérum humain, la tobramycine et l'albumine étant dans un rapport compris entre 20:1 et 28:1.

## Patentansprüche

1. Verfahren zur Messung der Menge an freiem Hapten in einer Probe unter Anwendung eines kompetitiven Immunpräzipitations-Assays, welches Verfahren umfasst die Schritte der Herstellung einer Lösung, die das freie Hapten, ein Hapten-Träger-Konjugat und Antikörper enthält, die mit dem freien Hapten und dem Konjugat eine Bindung eingehen, wobei die Trübung der Lösung in Abhängigkeit von der Menge des freien Haptens und des Verhältnisses von Hapten zu Träger in dem Konjugat in der Lösung in einem messbaren Grad variiert, und der Bestimmung der Lösungstrübung durch Analyse der Strahlung bei einer Wellenlänge innerhalb des Bereiches von 280 bis 600 nm, die direkt durch die Lösung geleitet wird.

2. Verfahren nach Anspruch 1, worin das Konjugat an Human-Serumalbumin kovalent gebundenes Gentamicin mit einem Gentamicin:Albumin-Verhältnis von 17:1 bis 24:1 ist.

3. Verfahren nach Anspruch 1, worin das Konjugat an Human-Serumalbumin kovalent gebundenes Phenytoin mit einem Phenytoin:Albumin-Verhältnis von 12:1 bis 40:1 ist.

4. Verfahren nach Anspruch 1, worin das Konjugat an Human-Serumalbumin kovalent gebundenes Phenobarbital mit einem Phenobarbital:Albumin-Verhältnis von 15:1 bis 30:1 ist.

5. Verfahren nach Anspruch 1, worin das Konjugat an Human-Serumalbumin kovalent gebundenes Theophyllin mit einem Theophyllin:Albumin-Verhältnis von 10:1 bis 20:1 ist.

6. Verfahren nach Anspruch 1, worin das Konjugat an Human-Serumalbumin kovalent gebundenes Trobamycin mit einem Trobramycin:Albumin-Verhältnis von 20:1 bis 28:1 ist.

7. Hapten-Träger-Konjugat, geeignet zur Vervollständigung eines kompetitiven Immunpräzipitations-Assays zur Bestimmung der Menge an freiem Hapten in einer Probe, die ein Hapten-Träger-Konjugat mit einem solchen Hapten:Träger-Verhältnis enthält, dass beim Vermischen des Konjugats in Lösung mit dem freien Hapten und einem Antikörper, der mit dem freien Hapten und dem Konjugat eine Bindung eingeht, variierende Mengen an freiem Hapten Trübungsänderungen der Lösung hervorrufen, die durch Messen von Strahlung innerhalb eines Wellenlängenbereiches von 280 bis 600 nm, die direkt durch die Lösung geleitet wird, bestimmbar sind.

8. Hapten-Träger-Konjugat nach Anspruch 7, worin das Konjugat an Human-Serumalbumin kovalent gebundenes Gentamicin mit einem Gentamicin:Albumin-Verhältnis von 17:1 bis 24:1 ist.

9. Hapten-Träger-Konjugat nach Anspruch 7, worin das Konjugat an Human-Serumalbumin kovalent gebundenes Phenytoin mit einem Phenytoin:Albumin-Verhältnis von 12:1 bis 40:1 ist.

10. Hapten-Träger-Konjugat nach Anspruch 7, worin das Konjugat an Human-Serumalbumin kovalent gebundenes Phenobarbital mit einem Phenobarbital:Albumin-Verhältnis von 15:1 bis 30:1 ist.

11. Hapten-Träger-Konjugat nach Anspruch 7, worin das Konjugat an Human-Serumalbumin kovalent gebundenes Theophyllin mit einem Theophyllin:Albumin-Verhältnis von 10:1 bis 20:1 ist.

12. Hapten-Träger-Konjugat nach Anspruch 7, worin das Konjugat an Human-Serumalbumin kovalent gebundenes Tobramycin mit einem Tobramycin:Albumin-Verhältnis von 20:1 bis 28:1 ist.

Fig. 1.

0 079 962

Fig. 2.

Fig. 3.

Fig. 4.

Fig. 5.